# EUROPÄISCHE PATENTSCHRIFT

(11) **EP 3 559 863 B1**
(45) Veröffentlichungstag und Bekanntmachung des Hinweises auf die Patenterteilung: **11.03.2026**
(21) Anmeldenummer: 17818090.7
(22) Anmeldetag: 11.12.2017
(51) Int. Cl.: G06V 10/42, G06V 10/50, G06V 10/60, A45D 44/00, A61B 5/00, G06Q 30/06, A45D 7/00

(54) **ERMITTLUNG EINES WELLIGKEITSWERTS VON HAAREN**
DETERMINATION OF A WAVINESS INDEX OF HAIR
DÉTERMINATION D'UNE VALEUR D'ONDULATION DES CHEVEUX

(30) Priorität: 20.12.2016 DE 102016225678
(43) Veröffentlichungstag der Anmeldung: 30.10.2019
(73) Patentinhaber: Henkel AG & Co. KGaA, 40589 Düsseldorf (DE)
(72) Erfinder: MÜLLER, Burkhard, 40221 Düsseldorf (DE); LECHNER, Torsten, 40764 Langenfeld (DE)
(86) Internationale Anmeldenummer: PCT/EP2017/082235
(87) Internationale Veröffentlichungsnummer: WO 2018/114434

(56) Entgegenhaltungen:
- DE-A1- 102014 220 077
- JP-A- H04 105 045
- US-A1- 2014 313 302
- MCMULLEN R. ET AL: "Optical properties of hair: detailed examination of specular reflection patterns in various hair types", JOURNAL OF COSMETIC SCIENCE, vol. 55, no. 1, 1 January 2004 (2004-01-01), US, XP055810392, ISSN: 1525-7886
- N LEFAUDEUX ET AL: "New luster formula for the characterization of hair tresses using polarization imaging", JOURNAL OF COSMETIC SCIENCE, March 2009 (2009-03-01), United States, pages 153 - 169, XP055147578, Retrieved from the Internet <URL:http://www.ncbi.nlm.nih.gov/pubmed/19450417> DOI: 10.1111/j.1468-2494.2010.00534_7.x
- SVANERA MICHELE ET AL: "Figaro, hair detection and segmentation in the wild", 2016 IEEE INTERNATIONAL CONFERENCE ON IMAGE PROCESSING (ICIP), IEEE, 25 September 2016 (2016-09-25), pages 933 - 937, XP033016615, DOI: 10.1109/ICIP.2016.7532494

## Beschreibung

Die Erfindung betrifft ein Verfahren und eine Vorrichtung zum Ermitteln eines Welligkeitswerts von Haaren und ein Verfahren zum Ermitteln einer individuellen Haarbehandlungsempfehlung. Glänzendes, gesund aussehendes Haar kann ein wichtiges kosmetisches Ziel sein, welches mittels kosmetischer Produkte zur Glanzerhöhung erzielbar sein kann.

Eine Eignung eines Glanzerhöhungsprodukts für einen Haartyp kann von einer Haarform des Haars, z.B. ob das Haar glatt oder wellig ist, abhängig sein.

Nutzer wissen oft nicht, welche Glanzerhöhungsprodukte für ihren Haartyp (z.B. bzgl. ihrer Haarform) am besten geeignet sind, um glänzendes Haar zu erzeugen. Ein Grund dafür kann sein, dass ihnen keine Möglichkeit zur Verfügung steht, um objektiv ihre Haarform zu beurteilen. Eine Bewertung einer Welligkeit von Haar erfolgt bislang entweder subjektiv, d.h., mittels Inaugenscheinnahme, oder beispielsweise indirekt durch ein Anwenden von Surrogat-Methoden. Als eine solche Surrogat-Methode kann beispielsweise eine Kämmbarkeit des Haars ermittelt werden, was indirekt Rückschlüsse auf eine Glattheit/Welligkeit des Haars ermöglichen kann.

Eine solche Ermittlung der (z.B. quantifizierten) Kämmbarkeit kann relativ aufwändig in einem Labor durchgeführt werden, eignet sich jedoch nicht für einen Endverbraucher, und ist auch nicht mittels einer mobilen Anwendung nutzbar.

Ein weiterer Grund dafür, dass Nutzer oft nicht wissen, welche Glanzerhöhungsprodukte für sie geeignet sind, kann sein, dass die Produkte nicht mit Informationen dahingehend versehen sind, für welche Haarform sie geeignet sind.

Die Patentanmeldung JP H04 105045 A (POLA CHEM IND INC), 7. April 1992, und die Druckschrift "Optical properties of hair - Detailed examination of specular reflection patterns in various hair types" von R. McMullen und J.Jachowicz, J. Cosmet. Sci. 55, 29-47 (Januar/Februar 2004) beschreiben Verfahren zur Messung des Haarglanzes.

Es besteht somit ein Bedarf an einer quantitativen Bewertung einer Welligkeit von Haar, beispielsweise einer Frisur. Wünschenswert wäre dabei ferner eine einfache Anwendbarkeit ohne oder mit nur geringem apparativem Aufwand, so dass die Welligkeit des Haars beispielsweise auch von einem Nutzer ermittelt werden kann.

Ferner besteht ein Bedarf daran, eine Produktempfehlung für ein Glanzerhöhungsprodukt zu erhalten, welches für eine Haarform eines Nutzers geeignet ist.

In verschiedenen Ausführungsbeispielen wird ein Verfahren gemäß Anspruch 1 zum Ermitteln eines Welligkeitsgrads von Haaren, der z.B. als Welligkeitswert quantifiziert sein kann, bereitgestellt.

Der ermittelte Welligkeitswert kann in verschiedenen Ausführungsbeispielen genutzt werden, um ein für Haar mit dem ermittelten Welligkeitswert empfehlenswertes Produkt zur Glanzerhöhung zu ermitteln.

In verschiedenen Ausführungsbeispielen kann das Ermitteln des Welligkeitswerts genutzt werden, um einen Erfolg einer Formänderung des Haars, beispielsweise eines Glättungs- oder Lockungsvorgangs, zu ermitteln.

In verschiedenen Ausführungsbeispielen kann zum Ermitteln der Welligkeit von Haaren ausgenutzt werden, dass ein (z.B. digitales) Bild welliger Haare stärkere Hell-/Dunkel-Kontraste aufweist als ein Bild glatter Haare.

Ein digitales Bild, auf welchem Haare abgebildet sind, kann in verschiedenen Ausführungsbeispielen mittels einer Digitalkamera aufgenommen sein oder werden.

Während des Aufnehmens des Bildes können in verschiedenen Ausführungsbeispielen die Haare mit diffusem Licht beleuchtet werden oder sein. Damit können starke Reflexe, die von einer intensiven gerichteten Lichtquelle erzeugt werden könnten, und welche ein Unterscheiden von Haaren mit verschiedenen Welligkeitswerten anhand des Helligkeitsunterschieds erschweren könnten, vermieden oder gemildert werden oder sein.

In verschiedenen Ausführungsbeispielen kann das Verfahren mit nur geringem oder ohne apparativen Aufwand ausführbar sein. Beispielsweise kann das Verfahren mittels einer App auf einem Tablet oder einem Smartphone ausführbar sein. Das kann es einem Nutzer beispielsweise ermöglichen, auch ohne professionelle Unterstützung und ohne eine Haarprobe für eine Manipulation bereitstellen zu müssen, den Welligkeitswert seines Haars, z.B. seiner Frisur, zu bestimmen, beispielsweise unter Verwendung eines Smartphones oder Tablets zum Aufnehmen eines digitalen Bildes der Frisur und eines Ermittelns des Glättewerts mittels des Smartphones/Tablets, wobei das Smartphone/Tablet in verschiedenen Ausführungsbeispielen genutzt werden kann, um das Bild einer externen Datenverarbeitungsvorrichtung, z.B. einer Cloud, bereitzustellen und die dort ermittelten Ergebnisse zu empfangen und dem Nutzer anzuzeigen.

Ein Nutzer kann in verschiedenen Ausführungsbeispielen eine exakte, auf seine Haarwelligkeit abgestimmte Produktempfehlung, z.B. zur Erhöhung von Haarglanz, bekommen, ohne eine eigene Haarbeurteilung vornehmen zu müssen, sondern beispielsweise einfach, indem er eines oder mehrere Bilder von seinen Haaren mit seinem Smartphone aufnimmt oder aufnehmen lässt und diese mittels einer App zum Ermitteln einer Haarbehandlung, insbesondere eines Haarpflegeprodukts, auswerten lässt.

In verschiedenen Ausführungsbeispielen wird ein Verfahren zum Ermitteln eines Welligkeitswerts von Haaren bereitgestellt. Das Verfahren kann aufweisen ein Ermitteln und/oder Festlegen mindestens eines Haaruntersuchungsbereichs in mindestens einem digitalen Bild, in welchem Haare abgebildet sind, ein Ermitteln einer Mehrzahl von unterschiedlichen Helligkeitswerten in dem Haaruntersuchungsbereich, und ein Ermitteln des Welligkeitswerts von Haaren basierend auf der Mehrzahl von Helligkeitswerten.

In verschiedenen Ausführungsbeispielen kann das Ermitteln einer Mehrzahl von Helligkeitswerten ein Ermitteln eines minimalen Helligkeitswerts und eines maximalen Helligkeitswerts aufweisen, wobei das Ermitteln des Welligkeitswerts von Haaren basierend auf der Mehrzahl von Helligkeitswerten ein Ermitteln eines Quotienten des maximalen Helligkeitswerts und des minimalen Helligkeitswerts aufweisen kann.

In verschiedenen Ausführungsbeispielen kann das Ermitteln einer Mehrzahl von Helligkeitswerten ein Ermitteln einer Helligkeitsverteilung aufweisen, wobei das Ermitteln des Welligkeitswerts von Haaren basierend auf der Mehrzahl von Helligkeitswerten ein Ermitteln eines Werts für eine Breite der Helligkeitsverteilung aufweisen kann.

Das Verfahren weist ferner ein Aufnehmen des mindestens einen digitalen Bildes auf.

Das Verfahren weist ferner während des Aufnehmens des mindestens einen digitalen Bildes ein Beleuchten des aufgenommenen Haars mit diffusem Licht auf.

Das Aufnehmen des mindestens einen digitalen Bildes weist ein Anordnen einer Kamera zum Aufnehmen des digitalen Bildes derart auf, dass ein Winkel zwischen einer Hauptrichtung, aus der das diffuse Licht das Haar beleuchtet, und einer optischen Achse der Kamera einen Winkel von mindestens 75° aufweist.

In verschiedenen Ausführungsbeispielen kann das Aufnehmen des mindestens einen digitalen Bildes ein Aufnehmen einer Mehrzahl von digitalen Bildern aufweisen, wobei auf jedem Bild der Mehrzahl von digitalen Bildern Haare aus einem unterschiedlichen Bereich einer Frisur dargestellt sein können.

In verschiedenen Ausführungsbeispielen kann das Ermitteln des Welligkeitswerts von Haaren ein Ermitteln eines gemeinsamen Frisur-Welligkeitswerts basierend auf der Mehrzahl von Helligkeitswerten aus der Mehrzahl von digitalen Bildern aufweisen.

In verschiedenen Ausführungsbeispielen kann das Festlegen des mindestens einen Haaruntersuchungsbereichs ein Festlegen von Position und/oder Form und/oder Größe des Haaruntersuchungsbereichs aufweisen.

In verschiedenen Ausführungsbeispielen kann das Ermitteln und/oder Festlegen mindestens eines Haaruntersuchungsbereichs ein Ermitteln eines Haardarstellungsbereichs, in welchem die Haare im digitalen Bild abgebildet sind, und ein Festlegen zumindest eines Teils des Haardarstellungsbereichs als den mindestens einen Haaruntersuchungsbereich aufweisen.

In verschiedenen Ausführungsbeispielen kann der mindestens eine Haaruntersuchungsbereich den gesamten Haardarstellungsbereich aufweisen.

In verschiedenen Ausführungsbeispielen kann das digitale Bild in einem Farbraum parametrisiert sein, der eine Helligkeit als einen Parameter aufweist.

In verschiedenen Ausführungsbeispielen kann das Verfahren ferner ein Darstellen des ermittelten Ergebnisses aufweisen.

In verschiedenen Ausführungsbeispielen wird eine Vorrichtung zum Ermitteln eines Welligkeitswerts von Haaren bereitgestellt. Die Vorrichtung kann eine Datenverarbeitungsvorrichtung und eine Anzeigevorrichtung aufweisen, wobei die Vorrichtung eingerichtet sein kann, das Verfahren zum Ermitteln eines Welligkeitswerts von Haaren gemäß verschiedenen Ausführungsbeispielen auszuführen.

In verschiedenen Ausführungsbeispielen wird ein Verfahren zum Ermitteln einer individuellen Haarbehandlungsempfehlung bereitgestellt. Das Verfahren kann ein Ermitteln eines Welligkeitswerts von Haaren gemäß verschiedenen Ausführungsbeispielen und ein Ermitteln einer Haarbehandlung, insbesondere eines Haarpflegeprodukts, basierend auf dem ermittelten Welligkeitswert aufweisen.

In verschiedenen Ausführungsbeispielen kann die Haarbehandlung eine Haarbehandlung zur Glanzerhöhung aufweisen. In verschiedenen Ausführungsbeispielen kann das Haarpflegeprodukt ein Haarpflegeprodukt zur Glanzerhöhung aufweisen.

In verschiedenen Ausführungsbeispielen kann der Welligkeitswert einen Wert aus einer mehrstufigen Welligkeitsskala aufweisen, die Welligkeiten von sehr wenig bis sehr stark zugeordnet sein kann, wobei die Haarbehandlung, insbesondere das Haarpflegeprodukt, zur Glanzerhöhung eines sein kann aus einer Mehrzahl von Haarbehandlungen, insbesondere Haarpflegeprodukten, zur Glanzerhöhung, wobei jedem der Mehrzahl von Haarbehandlungen, insbesondere Haarpflegeprodukten, zur Glanzerhöhung eine Glanzerhöhungsstufe zugeordnet sein kann, und wobei das Ermitteln einer Haarbehandlung, insbesondere eines Haarpflegeprodukts, basierend auf dem ermittelten Welligkeitswert ein Ermitteln einer Haarbehandlung, insbesondere eines Haarpflegeprodukts, mit einer höheren Glanzerzeugungsstufe für Haar mit einem höheren Welligkeitswert aufweisen kann.

Ausführungsbeispiele der Erfindung sind in den Figuren dargestellt und werden im Folgenden näher erläutert.

### Es zeigen

- Figuren 1A: ein Bild, auf dem Haare dargestellt sind, zur Verwendung bei einem Verfahren zum Ermitteln eines Welligkeitswerts von Haaren gemäß verschiedenen Ausführungsbeispielen und ggf. bei einem Verfahren zum Ermitteln einer individuellen Haarbehandlungsempfehlung gemäß verschiedenen Ausführungsbeispielen;
- Figuren 1B und 1C: vergrößerte Haaruntersuchungsbereiche des Bilds aus Figur 1;
- Figur 2A: die vergrößerten Haaruntersuchungsbereiche aus Figur 1B zusammen mit eine Markierung eines maximalen und eines minimalen Helligkeitswerts gemäß verschiedenen Ausführungsbeispielen;
- Figur 2B: Helligkeitsverteilungen der Haaruntersuchungsbereiche aus Figur 2A gemäß verschiedenen Ausführungsbeispielen;
- Figuren 3A und 3B: schematische Darstellungen einer Anordnung zum Ausführen eines Verfahrens zum Ermitteln eines Welligkeitswerts von Haaren gemäß verschiedenen Ausführungsbeispielen;
- Figur 4: ein Flussdiagramm eines Verfahrens zum Ermitteln eines Welligkeitswerts von Haaren gemäß verschiedenen Ausführungsbeispielen;
- Figur 5: ein Flussdiagramm eines Verfahrens zum Ermitteln eines Haarpflegeprodukts gemäß verschiedenen Ausführungsbeispielen; und
- Figur 6: eine graphische Darstellung einer Vorrichtung zum Ermitteln eines Welligkeitswerts von Haaren gemäß verschiedenen Ausführungsbeispielen.

In der folgenden ausführlichen Beschreibung wird auf die beigefügten Zeichnungen Bezug genommen, die Teil der vorliegenden Anmeldung bilden und in denen zur Veranschaulichung spezifische Ausführungsformen gezeigt sind, in denen die Erfindung ausgeübt werden kann. In dieser Hinsicht wird Richtungsterminologie wie etwa "oben", "unten", "vorne", "hinten", "vorderes", "hinteres", usw. mit Bezug auf die Orientierung der beschriebenen Figur(en) verwendet. Da Komponenten von Ausführungsformen in einer Anzahl verschiedener Orientierungen positioniert werden können, dient die Richtungsterminologie zur Veranschaulichung und ist auf keinerlei Weise einschränkend. Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche definiert.

Unter einem digitalen Bild kann hierin ein Datenpaket verstanden werden, welches von einem Datenverarbeitungssystem als zweidimensionale (flächige) Anordnung von Bildpunkten (auch als Pixel bezeichnet) darstellbar ist, beispielsweise in einem Koordinatensystem, welches eine x-Achse und eine y-Achse aufweist, wobei jeder Bildpunkt zumindest eine Bildposition als x,y-Koordinatenpaar und eine Intensitätsinformation aufweist, wobei die Intensitätsinformation beispielsweise als Farbe eines Pixels eines Monitors oder eines gedruckten Punktes eines ausgedruckten Bilds darstellbar ist. Die Intensitätsinformation kann sich bei einem Farbbild auf einzelne Farbkanäle beziehen. Das digitale Bild kann beispielsweise ein mit einer Digitalkamera aufgenommenes Foto oder ein Einzelbild einer mit einer digitalen Kamera aufgenommenen Videosequenz sein.

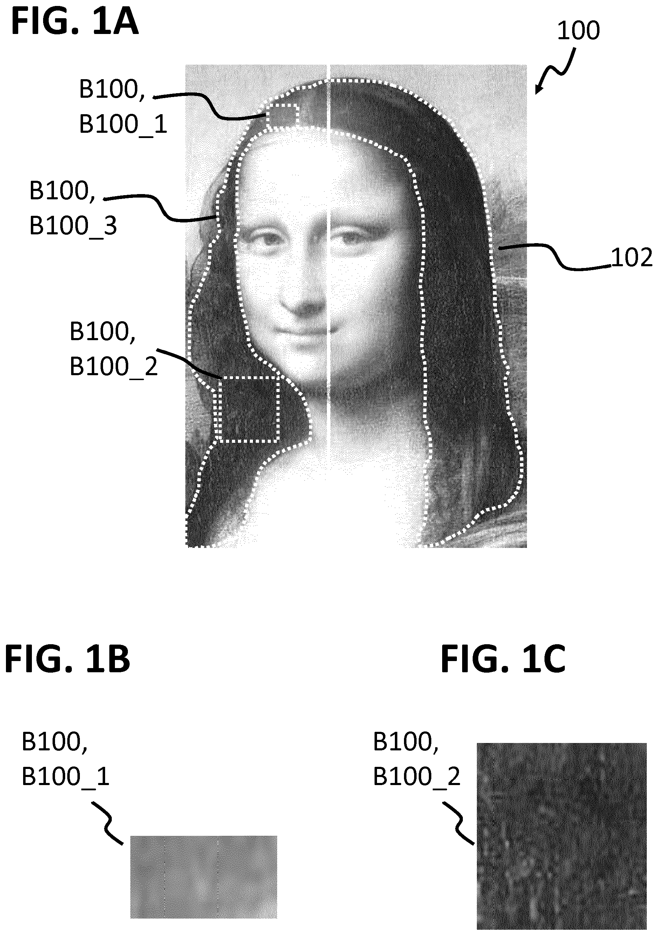

FIG. 1A zeigt ein Bild 100 eines Nutzers 102, in welchem Haare des Nutzers 102 abgebildet sind, zur Verwendung bei einem Verfahren zum Ermitteln eines Welligkeitswerts von Haaren gemäß verschiedenen Ausführungsbeispielen und ggf. bei einem Verfahren zum Ermitteln einer individuellen Haarbehandlungsempfehlung gemäß verschiedenen Ausführungsbeispielen.

Um das Verfahren zum Ermitteln einer Welligkeit von Haaren gemäß verschiedenen Ausführungsbeispielen anwenden zu können, kann das Bild 100 digital bereitgestellt sein. Das digitale Bild 100 kann beispielsweise einer Datenverarbeitungsvorrichtung bereitgestellt sein.

In verschiedenen Ausführungsbeispielen kann das digitale Bild 100 ein Bild 100, z.B. ein so genanntes Schwarzweißbild, sein, das lediglich Helligkeitsinformation aufweist, beispielsweise ein Graustufenbild, bei welchem Weiß einer größten Helligkeit zugeordnet sein kann, und schwarz einer geringsten Helligkeit.

In verschiedenen Ausführungsbeispielen kann das digitale Bild 100 ein Farbbild 100 sein, das in einem Farbraum (z.B. L*a*b*) mit einer Mehrzahl von Parametern codiert ist, wobei einer der Parameter eine Helligkeit sein kann. Für ein Ausführen des hierin beschriebenen Verfahrens, z.B. zum Ermitteln des Welligkeitswerts, kann eine Untersuchung des Bildes auf den Helligkeitsparameter beschränkt sein oder werden.

In verschiedenen Ausführungsbeispielen kann ein Bild U100 bereitgestellt werden, welches nicht digitalisiert ist. Das Bild U100 kann beispielsweise mittels Einscannens in das digitale Bild 100 umgewandelt werden.

In verschiedenen Ausführungsbeispielen kann ein Bild U100 bereitgestellt werden, welches in einem Farbraum parametrisiert ist, der keinen Helligkeitsparameter aufweist, z.B. RGB. Das Bild U100 kann beispielsweise mittels Transformierens in einen Farbraum, der einen Helligkeitsparameter aufweist umgewandelt werden, z.B. L*a*b*. Für eine nachfolgende Untersuchung zum Ermitteln der Welligkeit von Haaren kann dann lediglich der Helligkeitsparameter herangezogen werden und die weiteren Parameter (z.B. Farbton, Sättigung) ignoriert werden.

In verschiedenen Ausführungsbeispielen kann in dem Bild 100 mindestens ein Haaruntersuchungsbereich B100 bestimmt werden oder sein. Hier dargestellt sind drei Haaruntersuchungsbereiche B100_1, B100_2, B100_3. Die Haaruntersuchungsbereiche B100_1 und B100_2 sind außerdem vergrößert in Figuren 1B bzw. 1C dargestellt.

Um einen einem Betrachter unmittelbar ersichtlichen Unterschied in der Welligkeit des Haars der Haaruntersuchungsbereiche B100_1 aus FIG. 1B (relativ glatt) und B100_2 aus FIG. 1C (relativ wellig) zu quantifizieren, kann in verschiedenen Ausführungsbeispielen auf das digitale Bild 100 ein Bildverarbeitungsverfahren angewendet werden, mittels dessen beispielsweise jeder der Haaruntersuchungsbereiche B100_1, B100_2 getrennt untersucht werden kann.

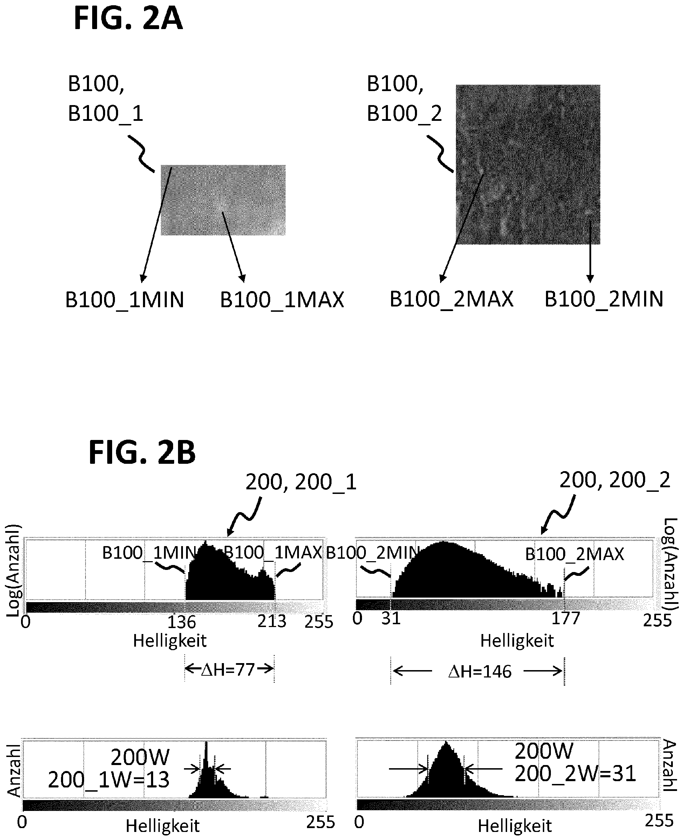

Figur 2A zeigt die vergrößerten Haaruntersuchungsbereiche B100_1, B100_2 aus Figur 1B mit einer Markierung von jeweils einem maximalen Helligkeitswert MAX (hier B100_1MAX für den Haaruntersuchungsbereich B100_1 bzw. B100_2MAX für den Haaruntersuchungsbereich B100_2) und einem minimalen Helligkeitswert MIN (hier B100_1MIN für den Haaruntersuchungsbereich B100_1 bzw. B100_2MIN für den Haaruntersuchungsbereich B100_2) gemäß verschiedenen Ausführungsbeispielen.

Figur 2B zeigt Helligkeitsverteilungen 200_1, 200_2 der Haaruntersuchungsbereiche B100_1 bzw. B100_2 aus Figuren 1A, 1B, 1C und 2A gemäß verschiedenen Ausführungsbeispielen. Dabei zeigt die obere Graphik jeweils die Helligkeitsverteilung in logarithmischer Darstellung der vertikalen Achse, die untere Graphik zeigt jeweils die Helligkeitsverteilung in linearer Darstellung der vertikalen Achse.

In verschiedenen Ausführungsbeispielen kann der Bereich des Bildes 100, in dem Haare (z.B. Haupthaar, also beispielsweise ohne Augenbrauen, Bart, usw.) dargestellt sind, als Haardarstellungsbereich bezeichnet werden. Der Haardarstellungsbereich kann eine Mehrzahl von Bildpunkten des digitalen Bildes 100 aufweisen, welche das Haar 102 abbilden und welche eine zusammenhängende oder aus einer Mehrzahl von Einzelflächen bestehende Fläche bilden können. Eine Ebene, in welcher der Haarbereich angeordnet sein kann, kann beispielsweise durch die x-Achse und die y-Achse des digitalen Bildes bestimmt sein.

In verschiedenen Ausführungsbeispielen kann das digitale Bild 100 zusätzlich zu dem Haardarstellungsbereich noch weitere Darstellungsbereiche aufweisen, in welchen beispielsweise Objekte, Körperteile, usw. dargestellt sein können. Der Haaruntersuchungsbereich B100 kann in verschiedenen Ausführungsbeispielen so gewählt sein, dass kein Teil der sonstigen Darstellungsbereiche in den Haaruntersuchungsbereich B100 fällt.

In verschiedenen Ausführungsbeispielen kann der Haaruntersuchungsbereich B100 einen Teilbereich des Haardarstellungsbereichs aufweisen. Die in FIG. 1A bis FIG. 2B dargestellten beispielhaften Haaruntersuchungsbereiche B100_1, B100_2 weisen jeweils einen Teilbereich des Haardarstellungsbereichs auf.

In verschiedenen Ausführungsbeispielen kann der Haaruntersuchungsbereich B100 den gesamten Haardarstellungsbereich B100 aufweisen. Der in FIG. 1A dargestellte beispielhafte Haaruntersuchungsbereich B100_3 weist den gesamten Haardarstellungsbereich auf.

In verschiedenen Ausführungsbeispielen, z.B. wenn im digitalen Bild 100 nur Haare dargestellt sind, kann der Haaruntersuchungsbereich B100 im Wesentlichen den gesamten Bereich des digitalen Bildes 100 aufweisen. In dem Fall kann der Haaruntersuchungsbereich B100 rechteckig, z.B. quadratisch, geformt sein.

In verschiedenen Ausführungsbeispielen kann der Haaruntersuchungsbereich B100 eine beliebige Form aufweisen, beispielsweise kann der Haaruntersuchungsbereich B100 rechteckig (z.B. quadratisch), dreieckig, polygonal mit einer anderen Eckenzahl als drei oder vier, elliptisch, rund oder beliebig anders geformt sein.

In verschiedenen Ausführungsbeispielen, in welchen der Haaruntersuchungsbereich B100 den gesamten Haardarstellungsbereich aufweist, kann folglich der Haaruntersuchungsbereich B100 eine ein- oder mehrteilige Fläche aufweisen, welche für eine nachfolgende Analyse, z.B. für ein Ermitteln von Helligkeitswerten, auch im Fall der mehrteiligen Fläche als ein einziger Haaruntersuchungsbereich B100 untersucht werden kann.

In verschiedenen Ausführungsbeispielen kann der mindestens eine Haaruntersuchungsbereich B100 eine Mehrzahl von Haaruntersuchungsbereichen B100 aufweisen, siehe z.B. in FIG. 1A die Haaruntersuchungsbereiche B100_1, B100_2 und B100_3. In verschiedenen Ausführungsbeispielen kann bei einer nachfolgenden Analyse, z.B. beim Ermitteln einer Mehrzahl unterschiedlicher Helligkeitswerte, jeder Haaruntersuchungsbereich B100_1, B100_2 bzw. B100_3 von der Mehrzahl von Haaruntersuchungsbereichen B100_1, B100_2 bzw. B100_3 getrennt untersucht werden, d.h. für jeden der Mehrzahl von Haaruntersuchungsbereichen B100_1, B100_2 bzw. B100_3 kann die Mehrzahl unterschiedlicher Helligkeitswerte separat ermittelt werden.

In verschiedenen Ausführungsbeispielen kann das Ermitteln und/oder Festlegen des mindestens einen Haaruntersuchungsbereichs B100 das Ermitteln des Haardarstellungsbereichs in dem Bild 100, beispielsweise mittels bekannter Verfahren, und ein Festlegen des mindestens einen Haaruntersuchungsbereichs B100 aufweisen.

Anders ausgedrückt können gemäß verschiedenen Ausführungsbeispielen ein Gesicht des Nutzers, d.h. alle Gesichtsflächen, die keine bzw. wenig Haare enthalten, und der Hintergrund in einem ersten Schritt ausgeblendet werden.

Beispielsweise kann das Festlegen des mindestens einen Haaruntersuchungsbereichs B100 bedeuten, dass, wie oben beschrieben, der gesamte Haardarstellungsbereich als der Haaruntersuchungsbereich B100 festgelegt wird, und/oder ein- oder mehrere Haaruntersuchungsbereich/e B100 können, beispielsweise automatisiert mittels der Datenverarbeitungsvorrichtung, z.B. unter Einbeziehung vorbestimmter Bedingungen, festgelegt werden. Beispielsweise kann eine Größe und/oder Anzahl der Haaruntersuchungsbereiche B100 vorgegeben werden, z.B. mittels eines Nutzers, und die Haaruntersuchungsbereiche B100 können dann automatisiert, z.B. mittels einer geeigneten Software, festgelegt werden, beispielsweise so, dass zwischen den Haaruntersuchungsbereichen B100 ein minimaler und/oder maximaler Abstand gewahrt bleibt, dass der Haardarstellungsbereich möglichst gleichmäßig abgedeckt wird, o.ä.

Für die oben beschriebenen Ermittlungen kann in verschiedenen Ausführungsbeispielen eine Software genutzt werden. Dabei kann jede Software genutzt werden, die eine oben beschriebene Funktionalität bereitstellt. In verschiedenen Ausführungsbeispielen, z.B. in einem Fall, dass zum Ausführen des Verfahrens zum Ermitteln eines Welligkeitswerts von Haaren ein Smartphone oder ein Tablet genutzt wird, kann die Software als eine App bereitgestellt sein.

In FIG. 1A sind im Bild 100 (zusätzlich zu dem den gesamten Haardarstellungsbereich aufweisenden Haaruntersuchungsbereich B100_3) zwei verschieden positionierte Haaruntersuchungsbereiche, ein erster Haaruntersuchungsbereich B100_1 und ein zweiter Haaruntersuchungsbereich B100_2, dargestellt.

In verschiedenen Ausführungsbeispielen können die beiden Haaruntersuchungsbereiche B100_1, B100_2 getrennt voneinander untersucht werden, z.B. derart, dass für jeden der Haaruntersuchungsbereiche B100_1, B100_2 eine eigene Mehrzahl von Helligkeitswerten ermittelt wird und daraus für jeden der Haaruntersuchungsbereiche B100_1, B100_2 ein eigener Welligkeitswert ermittelt wird.

In verschiedenen Ausführungsbeispielen kann die Mehrzahl verschieden positionierter Haaruntersuchungsbereiche B100_1, B100_2 genutzt werden, um einen Anhaltspunkt für eine Verteilung von welligem und/oder glattem Haar in einer Frisur zu erhalten. Beispielsweise können bei einer Frisur die Haare nahe der Kopfhaut (im Beispiel der Haaruntersuchungsbereich B100_1) glatt sein, während die Haare nahe den Spitzen (im Beispiel der Haaruntersuchungsbereich B100_2) gewellt sein können.

In verschiedenen Ausführungsbeispielen kann zum Ermitteln eines Welligkeitswerts von Haaren, was ein Quantifizieren einer Welligkeit von Haaren sein kann, ein Unterschied in einem Erscheinungsbild welligen Haars im Vergleich zu glattem Haar genutzt werden, welcher sich auch in einer, z.B. zweidimensionalen, Abbildung zeigt.

Bei welligen Haaren können Teilbereiche der Haare derart zueinander angeordnet sein, z.B. mit einem Winkelversatz zueinander und/oder teilweise derart übereinander angeordnet, dass sich keine zusammenhängende Fläche (oder höchstens kleine Flächenbereiche) nebeneinanderliegender Haare ergibt. Dies kann zur Folge haben, dass selbst bei Beleuchten der Haare mit diffusem Licht beim welligen Haar ein Schattenwurf von Haaren auf darunter-/dahinterliegende Haare stattfindet, wohingegen beim glatten Haar ein solcher Schattenwurf nicht oder in wesentlich geringerem Ausmaß auftreten kann.

Helligkeitswerte der dargestellten Haare können somit bei den welligen Haaren eine größere Heterogenität aufweisen als bei den glatten Haaren.

Mittels Untersuchens der Helligkeitswerte der dargestellten Haare, z.B. mittels Ermittelns einer Mehrzahl von Helligkeitswerten der Haare und einem Auswerten der Helligkeitswerte, z.B. mittels ins-Verhältnis-Setzen (d.h. Quotientenbildung), Differenzbildung, Ableitung statistischer Parameter o.ä., wird ein Maß für die Heterogenität der Helligkeitswerte der dargestellten Haare ermittelt.

In verschiedenen Ausführungsbeispielen, beispielsweise im Vergleich mit bereitgestellten Referenzwerten und/oder im Vergleich von Helligkeitswerten für verschiedene Haaruntersuchungsbereiche B100 miteinander, kann ein absoluter und/oder relativer Welligkeitswert für die Haare ermittelt werden, z.B. für die Haare in dem Haaruntersuchungsbereich B100 und/oder für eine Frisur.

In verschiedenen Ausführungsbeispielen kann das Ermitteln einer Mehrzahl von Helligkeitswerten ein Ermitteln eines minimalen Helligkeitswerts MIN und eines maximalen Helligkeitswerts MAX aufweisen.

Das Ermitteln des Welligkeitswerts von Haaren basierend auf der Mehrzahl von Helligkeitswerten kann in verschiedenen Ausführungsbeispielen ein Ermitteln eines Helligkeitsverhältnisses (eines Quotienten) HV = MAX / MIN zwischen dem maximalen Helligkeitswert MAX und dem minimalen Helligkeitswert MIN aufweisen.

In FIG. 2B sind beispielhaft eine erste Helligkeitsverteilung 200_1 für den ersten Haaruntersuchungsbereich B100_1 und eine zweite Helligkeitsverteilung 200_2 für den zweiten Haaruntersuchungsbereich B100_2 dargestellt, in der oberen Abbildung in Richtung der vertikalen Achse logarithmisch skaliert, in der unteren Abbildung in Richtung der vertikalen Achse linear skaliert. Absolutwerte der y-Achse können irrelevant sein und sind weggelassen. Beispielsweise können y-Achsen-Werte auf einen Maximalwert von 1 normiert sein.

Die Darstellungen sind lediglich als eine Veranschaulichung des Verfahrens zu verstehen. In verschiedenen Ausführungsbeispielen kann beispielsweise beim Ermitteln des maximalen Helligkeitswerts MAX und des minimalen Helligkeitswerts MIN auf ein Ermitteln der Helligkeitsverteilung verzichtet werden, sondern stattdessen können, beispielsweise mittels einer Datenverarbeitungsvorrichtung, z.B. mittels eines Smartphones, eines Tablets, eines Laptops oder sonstigen Computers, auf welchen beispielsweise eine Software, z.B. eine App, installiert sein kann, direkt der maximale Helligkeitswert MAX und der minimale Helligkeitswert MIN ermittelt werden.

In verschiedenen Ausführungsbeispielen kann dem Helligkeitsverhältnis ein Welligkeitswert zugeordnet werden, beispielsweise mittels Vergleichens mit Referenzwerten. Die Referenzwerte können vorab, z.B. mittels empirischer Daten, erstellt worden sein und beispielsweise als Referenzdatenbank für die Datenverarbeitungsvorrichtung abrufbar sein, z.B. in einem Speicher der Datenverarbeitungsvorrichtung gespeichert.

Die Referenzdaten können beispielsweise eine mehrstufige Welligkeitsskala, z.B. mit mindestens zwei Welligkeitswerten (z.B. von "sehr gering" bis "sehr stark"), z.B. drei bis fünf Welligkeitswerten, z.B. maximal 20 Welligkeitswerten, aufweisen, welche für jeden der Welligkeitswerte der Welligkeitsskala mindestens einen Messwert aufweist, der mittels der ermittelten Mehrzahl von verschiedenen Helligkeitswerten ermittelbar ist, z.B. das Helligkeitsverhältnis.

Eine bespielhafte Zuordnung von Helligkeitsverhältnissen zu Welligkeitswerten ist in der folgenden Tabelle angegeben:

| Hell/Dunkel-Verhältnis HV | Haarwelligkeit (Welligkeitswert) |
|---|---|
| ≤1,3 | sehr gering |
| >1,3 - 3,0 | mäßig |
| >3,0 - 10,0 | stark |
| >10,0 | sehr stark |

In verschiedenen Ausführungsbeispielen kann das Helligkeitsverhältnis ferner genutzt werden, um einen relativen Welligkeitswert zu ermitteln, beispielsweise mittels Vergleichens der Helligkeitsverhältnisse für verschiedene Haaruntersuchungsbereiche miteinander und/oder verschiedene Frisuren miteinander.

Das Vergleichen der Helligkeitsverhältnisse miteinander, beispielsweise mittels Bildens eines Quotienten der Helligkeitsverhältnisse, kann ermöglichen, die Welligkeit der verglichenen Haaruntersuchungsbereiche B100 bzw. Frisuren relativ zueinander zu beurteilen, z.B. welcher der Bereiche bzw. der Frisuren der/die Welligere ist. Bei einem Quotienten größer 1 ist der Bereich, dessen Helligkeitsverhältnis den Dividenden bildet, der Welligere, bei einem Quotienten kleiner 1 ist der Bereich, dessen Helligkeitsverhältnis den Divisor bildet, der Welligere. Ein Vergleichen von mehr als zwei Haaruntersuchungsbereichen B100 paarweise miteinander und/oder mit jeweils einem gemeinsamen Referenz-Haaruntersuchungsbereich kann es anhand verschieden großer Quotienten ermöglichen, eine Welligkeits-Reihenfolge zu ermitteln.

Im Beispiel in FIG. 2B weist der erste Haaruntersuchungsbereich B100_1 (in welchem das Haar glatt erscheint) einen minimalen Helligkeitswert von MIN = 136 und einen maximalen Helligkeitswert MAX von 213 auf. Daraus ergibt sich ein Helligkeitsverhältnis von HV = 213 / 136 = 1,57.

Der zweite Haaruntersuchungsbereich B100_2 (in welchem das Haar wellig erscheint) weist einen minimalen Helligkeitswert von MIN = 31 und einen maximalen Helligkeitswert MAX von 177 auf. Daraus ergibt sich ein Helligkeitsverhältnis von 177 / 31 = 5,71.

Die Absolutwerte der Helligkeitsverhältnisse HV zeigen, dass der Haaruntersuchungsbereich B100_2 mit dem Helligkeitsverhältnis von HV = 5,71 (verglichen mit HV = 1,57 für den Haaruntersuchungsbereich B100_1) das welligere Haar aufweist.

Allgemein können in verschiedenen Ausführungsbeispielen Helligkeitsverhältnisse in einem Bereich von etwa 1,1 bis etwa 100 zu erwarten sein, z.B. von etwa 1,3 bis etwa 20.

Ebenso zeigt sich anhand eines Verhältnisses der Helligkeitsverhältnisse von 5,71 / 1,57 = 3.6, dass im Haaruntersuchungsbereich des Dividenden (also des Haaruntersuchungsbereichs B100_2) das welligere Haar abgebildet ist.

Das Ermitteln des Welligkeitswerts von Haaren basierend auf der Mehrzahl von Helligkeitswerten kann in verschiedenen Ausführungsbeispielen ein Ermitteln einer Helligkeitsdifferenz ΔH = MAX - MIN zwischen dem maximalen Helligkeitswert MAX und dem minimalen Helligkeitswert MIN aufweisen.

In verschiedenen Ausführungsbeispielen können die in den Referenzdaten bereitgestellten Messwerte normiert sein, z.B. mittels einer Anzahl möglicher Helligkeitswerte (im Beispiel aus FIG. 2B sind das 256 Graustufen). Damit kann eine bessere Vergleichbarkeit der Referenzdaten mit Bildern ermöglicht sein, die eine Vielfalt von Helligkeitswerten aufweisen können, z.B. mehr oder weniger als 256 mögliche Helligkeitswerte.

Im Beispiel in FIG. 2B weist der erste Haaruntersuchungsbereich B100_1 (in welchem das Haar glatt erscheint) den minimalen Helligkeitswert von MIN = 136 und den maximalen Helligkeitswert MAX von 213 auf. Daraus ergibt sich eine Helligkeitsdifferenz von 77 und eine normierte Helligkeitsdifferenz von 0,3.

Der zweite Haaruntersuchungsbereich B100_2 (in welchem das Haar wellig erscheint) weist den minimalen Helligkeitswert von MIN = 31 und den maximalen Helligkeitswert MAX von 177 auf. Daraus ergibt sich eine Helligkeitsdifferenz von 146 und eine normierte Helligkeitsdifferenz von 0,57.

Die Absolutwerte der Helligkeitsdifferenzen (146 gegenüber 77 bzw. normiert 0,57 gegenüber 0,3) zeigen, dass der Haaruntersuchungsbereich B100_2 das welligere Haar aufweist.

Ebenso zeigt sich anhand eines Verhältnisses der Helligkeitsdifferenzen von 146/77 = 1.9, dass im Haaruntersuchungsbereich des Dividenden (also des Haaruntersuchungsbereichs B100_2) das welligere Haar abgebildet ist.

In verschiedenen Ausführungsbeispielen kann das Ermitteln einer Mehrzahl von Helligkeitswerten ein Ermitteln einer Helligkeitsverteilung 200 aufweisen, also ein Ermitteln, wie viele Bildelemente für alle möglichen Helligkeitswerte einen jeweiligen Helligkeitswert aufweisen.

In verschiedenen Ausführungsbeispielen kann das Ermitteln des Welligkeitswerts von Haaren basierend auf der Mehrzahl von Helligkeitswerten ein Ermitteln eines Werts 200W für eine Breite der Helligkeitsverteilung 200 aufweisen.

In verschiedenen Ausführungsbeispielen kann der ermittelten Breite der Helligkeitsverteilung 200 ein Welligkeitswert von einer Mehrzahl von Welligkeitswerten zugeordnet werden.

Für die Zuordnung des Welligkeitswerts zur Breite kann mutatis mutandis das gelten, was oben für die Helligkeitsdifferenz und eine Zuordnung eines Welligkeitswerts zur Helligkeitsdifferenz beschrieben ist, beispielsweise hinsichtlich eines Vergleichs der ermittelten Helligkeitsdifferenz mit Referenzdaten, und/oder hinsichtlich eines Vergleichs von ermittelten Helligkeitsdifferenzwerten, z.B. von verschiedenen Haaruntersuchungsbereichen B100, miteinander.

In verschiedenen Ausführungsbeispielen kann die Breite der Helligkeitsverteilung mindestens eins sein von einer vollen Halbwertsbreite, einer Standardabweichung, einer Äquivalentbreite, oder jedem anderen Maß, das eine Breite der Verteilung angibt.

Im Beispiel in FIG. 2B weist der erste Haaruntersuchungsbereich B100_1 (in welchem das Haar glatt erscheint) eine Breite von 200_1W = 13 auf, und eine normierte Breite von 0,05.

Der zweite Haaruntersuchungsbereich B100_2 (in welchem das Haar wellig erscheint) eine Breite von 200_2W = 31 auf, und eine normierte Breite von 0.12.

Die Absolutwerte der Breite zeigen, dass der Haaruntersuchungsbereich B100_2 das welligere Haar aufweist.

Ebenso zeigt sich anhand eines Verhältnisses der Breiten von 31/13 = 2,4, dass im Haaruntersuchungsbereich des Dividenden (also des Haaruntersuchungsbereichs B100_2) das welligere Haar abgebildet ist.

In verschiedenen Ausführungsbeispielen können anstelle der genannten Maße oder zusätzlich dazu beliebige andere Maße herangezogen werden, sofern sie geeignet sind, den Unterschied in den Helligkeitsverteilungen für glattes Haar und für gewelltes Haar zu quantifizieren. Beispielsweise kann, um das Ermitteln der Helligkeitsdifferenz robuster gegenüber Ausreißer-Werten zu machen, anstelle des Minimalwerts MIN einen Mittelwert aus mehreren der niedrigsten Werte, beispielsweise den zwei, drei oder vier niedrigsten Werten oder einem Prozentanteil, z.B. fünf bis fünfzehn, z.B. zehn Prozent, der niedrigsten Werte der Helligkeitsverteilung genutzt werden, und anstelle des Maximalwerts MAX einen Mittelwert aus beispielsweise den zwei, drei oder vier höchsten Werten oder einem Prozentanteil, z.B. fünf bis fünfzehn, z.B. zehn Prozent, der höchsten Werte der Helligkeitsverteilung.

In verschiedenen Ausführungsbeispielen können zwei oder mehr der ermittelten Werte kombiniert werden, beispielsweise eine Breite und ein Differenzwert, beispielsweise zu einem einzigen Wert, welcher dann beispielsweise mit den Datenbankwerten verglichen werden kann zum Ermitteln des Welligkeitswertes.

Mittels der im Haardarstellungsbereich verteilt angeordneten Haaruntersuchungsbereiche B100 können, beispielsweise in einem Fall, in welchem der Haardarstellungsbereich eine gesamte Frisur aufweist, ein Identifizieren welliger und/oder glatter Bereiche im Haardarstellungsbereich ermöglicht sein.

In verschiedenen Ausführungsbeispielen können, beispielsweise unter Verwendung bekannter Mittelungsverfahren, mehrere der Haaruntersuchungsbereiche B100 gemittelt werden.

In verschiedenen Ausführungsbeispielen kann ein Smartphone, ein Tablet, ein Laptop, oder ähnliches geeignet sein, um das Verfahren zum Ermitteln des Welligkeitswerts von Haaren auszuführen. In verschiedenen Ausführungsbeispielen braucht die Software nicht auf dem Smartphone, dem Tablet, dem Laptop usw. hergestellt zu werden. Es kann beispielsweise ausreichend sein, wenn das Smartphone o.ä. durch das Internet
mit einem Computer verbunden ist. In einem solchen Fall können die Berechnungen beispielsweise mittels des Computers ausgeführt werden, und das Ergebnis kann dem Smartphone/Tablet o.ä. bereitgestellt werden.

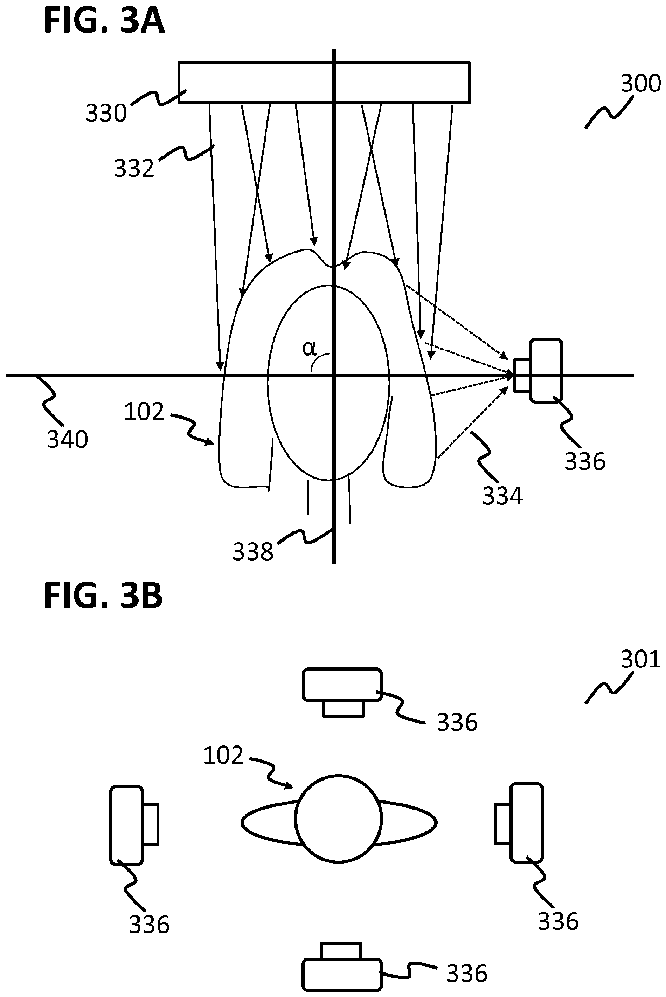

Figuren 3A und 3B zeigen schematische Darstellungen 300, 301 einer Anordnung zum Ausführen eines Verfahrens zum Ermitteln eines Welligkeitswerts von Haaren gemäß verschiedenen Ausführungsbeispielen.

In verschiedenen Ausführungsbeispielen kann eine Anordnung zum Ausführen des Verfahrens zum Ermitteln eines Welligkeitswerts von Haaren gemäß verschiedenen Ausführungsbeispielen eine Kamera 336, beispielsweise eine Digitalkamera, z.B. eine Kamera eines Devices, z.B. eines Smartphones, eines Tablets, eines Laptops, oder eine beliebige andere Digitalkamera aufweisen. In verschiedenen Ausführungsbeispielen kann auch eine analoge Kamera genutzt werden und die aufgenommenen Bilder können für ein Anwenden des Verfahrens zum Ermitteln eines Welligkeitswerts von Haaren digitalisiert und für das Verfahren bereitgestellt werden. In verschiedenen Ausführungsbeispielen kann die Kamera 336 eine digitale Videokamera aufweisen, wobei für das Ausführen des Verfahrens zum Ermitteln eines Welligkeitswerts von Haaren mindestens ein Einzelbild einer mittels der Videokamera aufgenommenen Videosequenz genutzt werden kann.

In verschiedenen Ausführungsbeispielen kann eine Anordnung zum Ausführen des Verfahrens zum Ermitteln eines Welligkeitswerts von Haaren gemäß verschiedenen Ausführungsbeispielen ferner eine Lichtquelle 330 aufweisen. Die Lichtquelle 330 kann so gestaltet und angeordnet sein, beispielsweise oberhalb eines Kopfes eines Nutzers 102, dass Haare des Nutzers 102 während eines Aufnehmens von Bildern, auf denen die Haare des Nutzers 102 abgebildet sind, von mittels der Lichtquelle 330 abgestrahltem Licht 332 beleuchtet werden. Die Lichtquelle 330 kann beispielsweise eine Beleuchtungsstärke in einem Bereich von etwa 100 Lux bis etwa 100000 Lux bereitstellen, z.B. von etwa 200 bis etwa 10000 Lux, z.B. von etwa 500 Lux bis etwa 2000 Lux.

In verschiedenen Ausführungsbeispielen kann das Licht 332 diffuses Licht 332 sein. Bei einer künstlichen Lichtquelle 330 kann dies beispielsweise mittels eines Diffusors, z.B. einer Streuscheibe, erreicht werden, oder beispielsweise mittels Beleuchtens einer Zimmerdecke, eines Reflexionsschirms o.ä., beispielsweise oberhalb des Nutzers 102, mit der Lichtquelle 330.

In verschiedenen Ausführungsbeispielen kann auch eine natürliche Lichtquelle 330, beispielsweise die Sonne, als die Lichtquelle 330 genutzt werden. In verschiedenen Ausführungsbeispielen kann eine direkte Sonneneinstrahlung auf die Haare des Nutzers dabei vermieden werden, beispielsweise mittels Anordnens des Nutzers in einem Halbschatten.

Mittels der diffusen Beleuchtung können in verschiedenen Ausführungsbeispielen Reflexionen (z.B. starke, schlaglichtartige Reflexionen) auf den Haaren, die einen sehr hohen und für Haar jeden Welligkeitswerts in etwa gleichen maximalen Helligkeitswert ergeben würden (was zu einer Fehlinterpretation des Welligkeitswerts, z.B. des Welligkeitsgrads, der Haare führen könnte) vermieden werden.

In verschiedenen Ausführungsbeispielen kann der Nutzer 102 so angeordnet sein, dass sein Kopf aufrecht angeordnet ist, d.h. seine Kopfoberseite nach oben zeigt, z.B. in Richtung zur Lichtquelle 330.

In verschiedenen Ausführungsbeispielen kann die Kamera 336 beim Aufnehmen des Bildes so angeordnet sein, dass mindestens ein Teil der Haare des Nutzers 102 auf dem Bild dargestellt sind. In verschiedenen Ausführungsbeispielen kann die Kamera 336 so angeordnet sein, dass sich ihr Objektiv auf einer Höhe von oberen Ohrpartien des Nutzers befindet und seine optische Achse 340 im Wesentlichen senkrecht zu einer Haaroberfläche der abgebildeten Haare ist. Damit kann erreicht werden, dass zum einen Haarbereiche des Nutzers 102 abgebildet werden, die hauptsächlich für einen Welligkeitseindruck, den eine Frisur des Nutzers 102 vermittelt, auf dem Bild abgebildet sein können, und andererseits, dass, zumindest bei einer Beleuchtung im Wesentlichen von oben, ein Winkel α zwischen einer Hauptbeleuchtungsrichtung 338 und der optischen Achse 340 der Kamera 336 einen Minimalwinkel nicht unterschreitet und einen Maximalwinkel nicht überschreitet. Der Winkel α kann beispielsweise in einem Bereich von etwa 75° bis etwa 105° liegen, z.B. von etwa 85° bis etwa 95°. Bei einer Anordnung der Kamera 336 mit einem anderen Winkel zur Hauptbeleuchtungsrichtung 338, beispielsweise mit der optischen Achse 340 im Wesentlichen parallel zur Hauptbeleuchtungsrichtung 338, kann möglicherweise eine Schattenbildung (und damit Helligkeitsunterschiede) auch bei welligem Haar auf dem Bild nicht oder weniger ausgeprägt erkennbar sein.

In verschiedenen Ausführungsbeispielen kann, wie in FIG. 2B dargestellt ist, Aufnahmen der Haare des Nutzers 102 aus verschiedenen Richtungen, d.h. von unterschiedlichen Haarbereichen, gemacht werden.

Beim Anfertigen nur einer Aufnahme kann diese in verschiedenen Ausführungsbeispielen die Haare des Nutzers am Hinterkopf abbilden, denn typischerweise weist der Hinterkopf die größte zusammenhängende Haarfläche auf, wenn der gesamte Kopf des Nutzers abgebildet wird. Allerdings kann in verschiedenen Ausführungsbeispielen auch ein anderer Haarbereich des Nutzers 102 als der einzige abgebildete Haarbereich verwendet werden.

In verschiedenen Ausführungsbeispielen können beim Aufnehmen von verschiedenen Haarbereichen ferner Aufnahmen von der linken und/oder der rechten Kopfseite gemacht werden, und/oder von jedem Haarbereich, der für das Ermitteln der Welligkeit der Haare des Nutzers 102 als nützlich erachtet wird.

In verschiedenen Ausführungsbeispielen kann bei einem Aufnehmen von drei oder mehr Bildern eine Anordnung der abgebildeten Bereiche symmetrisch sein, z.B. können die Aufnahmen der Kopfseite symmetrisch, d.h. mit gleichen Winkeln, zur Aufnahme des Hinterkopfs angeordnet sein (in FIG. 2B werden beispielsweise sowohl die rechte als auch die linke Kopfseite in einem Winkel von 90° zur Aufnahme des Hinterkopfs gemacht).

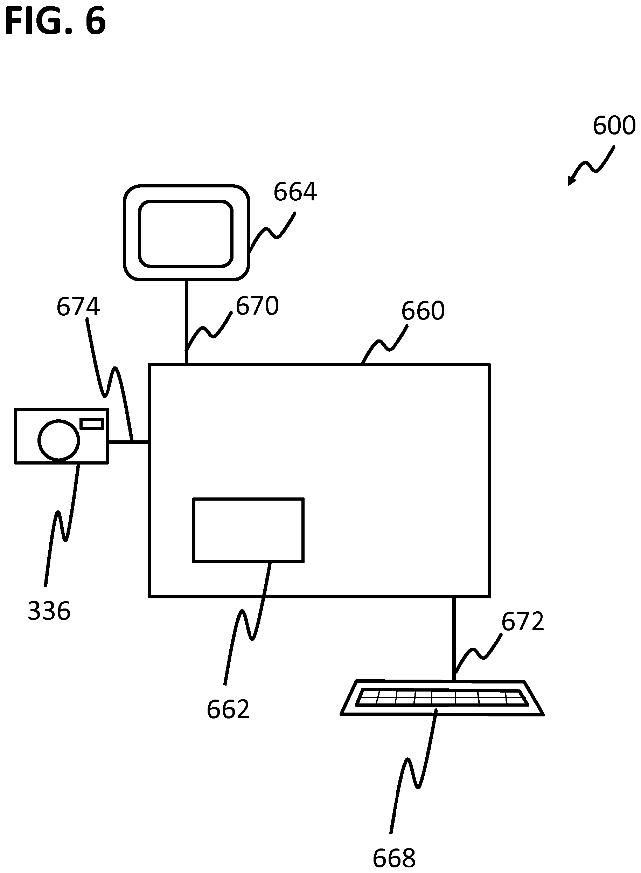

Für das weitere Ausführen des Verfahrens zum Ermitteln eines Welligkeitswerts von Haaren kann eine Datenverarbeitungsvorrichtung genutzt werden, wie sie beispielsweise oben und im Zusammenhang mit FIG. 6 beschrieben ist, z.B. ein Smartphone, ein Tablet o.ä.

In verschiedenen Ausführungsbeispielen können in den Bildern diejenigen Bereiche, in denen keine oder wenig Haare abgebildet sind, z.B. Gesichtsflächen und/oder Hintergrundbereiche, eliminiert werden, z.B. auf bekannte Weise, z.B. mittels Anwendens eines Morphing-Filters. Anders ausgedrückt kann der Haardarstellungsbereich, der überwiegend nur noch Haare enthalten kann, ermittelt werden, und der Bildbereich, der nicht der Haardarstellungsbereich ist, kann verworfen werden.

In verschiedenen Ausführungsbeispielen kann für jedes der verbliebenen Bildelemente ein Helligkeitswert ermittelt werden, d.h. ein Haaruntersuchungsbereich kann den gesamten Haardarstellungsbereich aufweisen. Alternativ kann in verschiedenen Ausführungsbeispielen mindestens ein Teilbereich des Haardarstellungsbereichs als Haaruntersuchungsbereich festgelegt werden, und das Ermitteln der Helligkeitswerte kann für den Teilbereich des Haardarstellungsbereichs ausgeführt werden.

In verschiedenen Ausführungsbeispielen kann für den Haaruntersuchungsbereich, z.B. den Haardarstellungsbereich, ein maximaler Helligkeitswert ermittelt werden, z.B. mittels Mittelns der hellsten 10% der Helligkeitswerte. Ferner kann für den Haaruntersuchungsbereich, z.B. den Haardarstellungsbereich, ein minimaler Helligkeitswert ermittelt werden, z.B. mittels Mittelns der dunkelsten 10% der Helligkeitswerte. Statt die hellsten/dunkelsten 10% zum Ermitteln des maximalen/minimalen Helligkeitswerts zu nutzen, können, z.B. wie oben beschrieben, andere Maße zum Ermitteln eines Helligkeitsunterschieds von Bildelementen im Bild genutzt werden, z.B. der höchste Helligkeitswert MAX und der niedrigste Helligkeitswert MIN, eine Breite der Helligkeitsverteilung, o.ä.

Aus dem maximalen Helligkeitswert und dem minimalen Helligkeitswert kann in verschiedenen Ausführungsbeispielen ein Helligkeitsverhältnis, d.h. ein Quotient der Helligkeitswerte, ermittelt werden, wobei es prinzipiell egal sein kann, welcher der Helligkeitswerte als ein Dividend genutzt wird und welcher als ein Divisor genutzt wird. Vorliegend kann, passend zur oben dargestellten Tabelle der Helligkeitsverhältnisse (auch als Hell/Dunkel-Verhältnis bezeichnet) der maximale Helligkeitswert der Dividend sein und der minimale Helligkeitswert der Divisor sein, so dass sich ein Helligkeitsverhältnis von >1 ergeben kann.

Entsprechend dem ermittelten Helligkeitsverhältnis (entsprechendes kann gelten, wenn andere Maße zum Ermitteln der Helligkeitsunterschiede verwendet werden) kann ein Welligkeitswert zugeordnet werden, beispielsweise ein Welligkeitswert von "sehr gering" bis "sehr stark".

In verschiedenen Ausführungsbeispielen kann basierend auf einem wie oben für verschiedene Ausführungsbeispiele beschrieben ermittelten Welligkeitswert eine individuelle Behandlungsempfehlung, z.B. ein für den Welligkeitswert geeignetes Haarpflegeprodukt, ermittelt werden.

Das Haarpflegeprodukt kann ein Produkt aus einer Gruppe von Haarpflegeprodukten aufweisen, von denen bekannt oder zu erwarten sein kann, dass ihre Eignung zu einer Anwendung bei Haar von einer Welligkeit des Haars abhängen kann, d.h., dass sie jeweils für Haar mit einem gegebenen Welligkeitswert besonders gut geeignet sein können.

In verschiedenen Ausführungsbeispielen kann die Gruppe von Haarpflegeprodukten z.B. Haarpflegeprodukte zur Glanzerhöhung von Haaren aufweisen.

Eine Zuordnung von Welligkeitswerten und geeigneten Haarpflegeprodukten, z.B. Glanzerhöhungsprodukten, kann beispielsweise als eine vorgegebene Datenbank, die beispielsweise auf empirischen Daten basieren kann, bereitgestellt sein, z.B. in einem Speicher der Datenverarbeitungsvorrichtung.

Die geeigneten Haarpflegeprodukte können dabei chemische Produktgruppen und/oder konkrete Produktnamen, unter welchen der Nutzer diese beispielsweise erwerben könnte, aufweisen.

Die Haarpflegeprodukte können den Welligkeitswerten in verschiedenen Stufen zugeordnet sein, oder umgekehrt kann die Gruppe von Haarpflegeprodukten in eine Mehrzahl von Pflegestufen, z.B. Glanzerhöhungsstufen, eingeteilt sein, und dazu können Welligkeitswerte, für welche die Haarpflegeprodukte der jeweiligen Glanzerhöhungsstufe geeignet sein können, festgelegt sein.

Für Glanzerhöhungsprodukte können für unterschiedliche Glanzerhöhungsstufen beispielsweise Inhaltsstoffe wie z.B. Dimethicone (mittlere Glanzerzeugung), Aprikosenkernöl (hohe Glanzerzeugung) und/oder Phenyltrimethicon (sehr starke Glanzerzeugung) verantwortlich sein. Glattes Haar kann von Natur aus mehr Glanz aufweisen als gewelltes Haar. Dies kann bei der Produktempfehlung berücksichtigt werden, indem für stärker wellige Haare stärker glanzerzeugende Produkte empfohlen werden.

Dementsprechend kann ein Nutzer in verschiedenen Ausführungsbeispielen eine exakte, auf seine Haarwelligkeit abgestimmte Produktempfehlung, z.B. zur Erhöhung von Haarglanz, bekommen, ohne eine eigene Haarbeurteilung vornehmen zu müssen, sondern beispielsweise einfach, indem er eines oder mehrere Bilder von seinen Haaren mit seinem Smartphone aufnimmt oder aufnehmen lässt und diese mittels einer App zum Ermitteln eines Haarpflegeprodukts auswerten lässt.

In verschiedenen Ausführungsbeispielen kann der Welligkeitswert einen Wert aus einer mehrstufigen Welligkeitsskala aufweisen, die Welligkeiten von sehr glatt bis sehr wellig zugeordnet sein kann, wobei das Haarpflegeprodukt zur Glanzerhöhung eines sein kann aus einer Mehrzahl von Haarpflegeprodukten zur Glanzerhöhung, wobei jedem der Mehrzahl von Haarpflegeprodukten zur Glanzerhöhung eine Glanzerhöhungsstufe zugeordnet sein kann, und wobei das Ermitteln eines Haarpflegeprodukts basierend auf dem ermittelten Welligkeitswert ein Ermitteln eines Haarpflegeprodukts mit einer höheren Glanzerzeugungsstufe für Haar mit einem höheren Welligkeitswert aufweisen kann.

In der nachfolgenden Tabelle ist eine beispielhafte Zuordnung von Produktempfehlungen für Haarwelligkeitswerte angegeben:

| Haarwelligkeit (Welligkeitswert) | Produktempfehlung |
|---|---|
| sehr gering | Produkt 1 für geringe Glanzerhöhung, enthaltend Trisiloxan (1%) |
| mäßig | Produkt 2 für mäßige Glanzerhöhung, enthaltend Dimethicon (1%) |
| stark | Produkt 3 für starke Glanzerhöhung, enthaltend Aprikosenkernöl (1%) |
| sehr stark | Produkt 4 für sehr starke Glanzerhöhung, enthaltend Phenyltrimethicon (1%) |

In der Tabelle können die Bezeichnungen "Produkt 1" usw. allgemeine Bezeichnungen für die jeweilige Produktgruppe sein, und/oder stellvertretend für jeweils einen oder mehrere Produktnamen, unter welchen der Nutzer das jeweilige Produkt kaufen könnte.

In verschiedenen Ausführungsbeispielen kann das Haarpflegeprodukt anstelle von oder zusätzlich zu einem Glanzerhöhungsprodukt ein anderes Haarpflegeprodukt aufweisen, dessen Eignung von einer Welligkeit des zu pflegenden Haars abhängig sein kann, beispielsweise eine Haarkur, ein/en Haarfestiger/-gel/-schaum, oder ähnliches.

Ein Ergebnis des Ermittelns der individuellen Behandlungsempfehlung, vorzugsweise des Haarpflegeprodukts zur Glanzerhöhung, kann dem Nutzer bereitgestellt werden, beispielsweise angezeigt oder auf andere Weise, beispielsweise per Sprachausgabe, zugänglich gemacht werden.

Es ist ferner bevorzugt, dass die individuelle Behandlungsempfehlung darin besteht, zum/vom Einsatz von Haarpflegeprodukten, die das Individuum anhand von QR-Codes, NFC-Chips, Barcodes oder RFID-Chips, identifiziert, zu- oder abzuraten.

Alternativ kann die individuelle Behandlungsempfehlung darin bestehen, zum Einsatz von Haarpflegeprodukten, die für den ermittelten Welligkeitswert individuell hergestellt werden, zu zuraten und einen Bestellvorgang, vorzugsweise durch Aufrufen einer Internetseite eines Herstellers von individuellen Haarpflegeprodukten, einzuleiten.

In verschiedenen Ausführungsbeispielen kann das Verfahren zum Bestimmen eines Welligkeitswerts von Haaren vor einem Anwenden eines Verfahrens zum Ändern einer Welligkeit von Haar, z.B. eines Glättungsverfahrens (z.B. mittels eines Conditioners, einer Haarkur, einer Dauerwelle, eines Relaxers, eines Straighteners und/oder eines Glätteisens) oder eines Locken-/Wellenerzeugungsverfahrens (z.B. mittels Dauerwelle oder Lockenwicklern) ermittelt werden. Damit kann beispielsweise ermittelt werden, ob bzw. an welchen Stellen eine Behandlung des Haars notwendig sein könnte.

Alternativ oder zusätzlich kann in verschiedenen Ausführungsbeispielen der Welligkeitswert von Haaren nach dem Anwenden des Verfahrens zum Ändern einer Welligkeit ermittelt werden. Damit kann beispielsweise eine Wirksamkeit des Verfahrens zum Ändern einer Welligkeit ermittelt werden. Dafür kann in verschiedenen Ausführungsbeispielen ein Verhältnis des Welligkeitswerts nach der Behandlung und des Welligkeitswerts vor der Behandlung ermittelt werden.

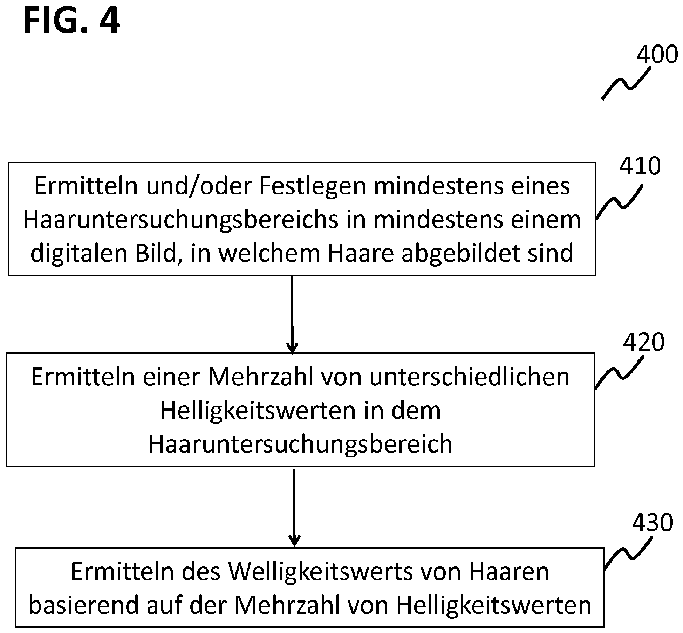

FIG. 4 zeigt ein Flussdiagramm 400 eines Verfahrens zum Ermitteln eines Welligkeitswerts von Haaren gemäß verschiedenen Ausführungsbeispielen.

In verschiedenen Ausführungsbeispielen kann das Verfahren ein Ermitteln und/oder Festlegen mindestens eines Haaruntersuchungsbereichs in mindestens einem digitalen Bild, in welchem Haare abgebildet sind, aufweisen (in 410), ein Ermitteln einer Mehrzahl von unterschiedlichen Helligkeitswerten in dem Haaruntersuchungsbereich (in 420), und ein Ermitteln des Welligkeitswerts von Haaren basierend auf der Mehrzahl von Helligkeitswerten (in 430).

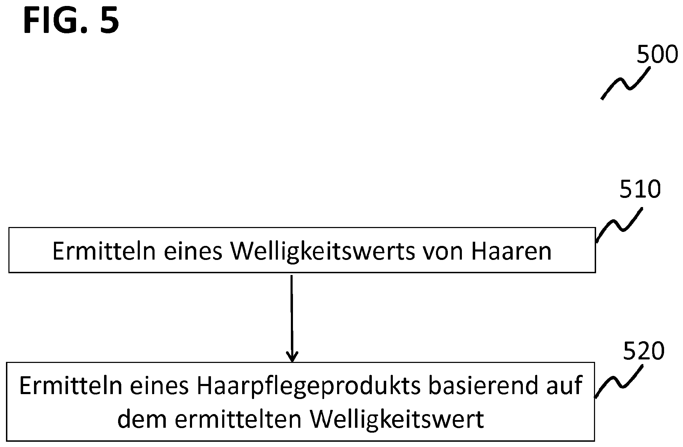

FIG. 5 zeigt ein Flussdiagramm 500 eines Verfahrens zum Ermitteln eines Welligkeitswerts von Haaren gemäß verschiedenen Ausführungsbeispielen.

In verschiedenen Ausführungsbeispielen kann das Verfahren ein Ermitteln eines Welligkeitswerts von Haaren aufweisen (in 510), beispielsweise gemäß verschiedenen oben beschriebenen Ausführungsbeispielen, und ein zum Ermitteln einer individuellen Behandlungsempfehlung, vorzugsweise eines Haarpflegeprodukts zur Glanzerhöhung, basierend auf dem ermittelten Welligkeitswert (in 520).

FIG. 6 zeigt eine graphische Darstellung einer Vorrichtung 600 zum Ermitteln eines Welligkeitswerts von Haaren gemäß verschiedenen Ausführungsbeispielen. Die Vorrichtung 600 kann ferner eingerichtet sein zum Ermitteln einer individuellen Behandlungsempfehlung, vorzugsweise eines Haarpflegeprodukts zur Glanzerhöhung, gemäß verschiedenen Ausführungsbeispielen.

In verschiedenen Ausführungsbeispielen kann die Vorrichtung 600 zum Ermitteln eines Welligkeitswerts von Haaren bzw. zum Ermitteln einer individuellen Behandlungsempfehlung, vorzugsweise eines Haarpflegeprodukts zur Glanzerhöhung, eine Datenverarbeitungsvorrichtung 660 aufweisen.

Die Datenverarbeitungsvorrichtung 660 kann beispielsweise einen Computer, ein Tablet, ein Smartphone, einen Laptop oder jede andere Datenverarbeitungsvorrichtung, welche geeignet ist, das Verfahren zum Ermitteln eines Welligkeitswerts von Haaren, zum Ermitteln eines Haarpflegeprodukts oder das Verfahren zum Ermitteln einer individuellen Behandlungsempfehlung gemäß verschiedenen Ausführungsbeispielen auszuführen, aufweisen. Vereinfachend wird die Datenverarbeitungsvorrichtung 660 hierin auch als Computer 660 bezeichnet. Die Datenverarbeitungsvorrichtung 660 kann einen Prozessor 662, beispielsweise einen Mikroprozessor, aufweisen.

In verschiedenen Ausführungsbeispielen kann die Vorrichtung 600 zum Ermitteln eines Welligkeitswerts von Haaren bzw. zum Ermitteln einer individuellen Behandlungsempfehlung, vorzugsweise eines Haarpflegeprodukts zur Glanzerhöhung, eine Anzeigevorrichtung 664 aufweisen.

Die Anzeigevorrichtung 664 kann beispielsweise einen Bildschirm eines Smartphones, eines PCs, eines Laptops oder einer sonstigen Vorrichtung 600 zum Ermitteln eines Welligkeitswerts von Haaren bzw. zum Ermitteln eines Haarpflegeprodukts aufweisen. Die Anzeigevorrichtung 664 kann beispielsweise genutzt werden, um Ergebnisse des Verfahrens zum Ermitteln eines Welligkeitswerts von Haaren bzw. zum Ermitteln einer individuellen Behandlungsempfehlung, vorzugsweise eines Haarpflegeprodukts zur Glanzerhöhung, darzustellen, Eingabeparameter für das Ausführen des Verfahrens zu erfragen, oder Ähnliches.

Die Anzeigevorrichtung 664 kann mittels einer ersten Datenverbindung 670 mit der Datenverarbeitungsvorrichtung 660 verbunden sein. Die Anzeigevorrichtung 664 kann mit der Datenverarbeitungsvorrichtung 660 mittels der ersten Datenverbindung 670 Daten austauschen. In einem Fall, dass die Vorrichtung 600 ein Smartphone, einen Tablet o.ä. aufweist, können die Anzeigevorrichtung 664 und die erste Datenverbindung 670 in der Vorrichtung 600 integriert sein.

In verschiedenen Ausführungsbeispielen kann die Vorrichtung 600 zum Ermitteln eines Welligkeitswerts von Haaren bzw. zum Ermitteln eines Haarpflegeprodukts eine Kamera 336 aufweisen.

Die Kamera 336 kann gemäß verschiedenen Ausführungsbeispielen eingerichtet sein, ein digitales Bild 100 von Haar 102, z.B. von Haar eines Nutzers, aufzunehmen, beispielsweise wie oben beschrieben.

Die mindestens eine Kamera 336 kann gemäß verschiedenen Ausführungsbeispielen eine digitale Fotokamera oder eine Videokamera, d.h. eine Kamera 104, welche eingerichtet sein kann, eine Mehrzahl von Einzelbildern als Zeitfolge aufzuzeichnen, aufweisen.

In verschiedenen Ausführungsbeispielen kann die Vorrichtung 600 zum Ermitteln eines Welligkeitswerts von Haaren bzw. zum Ermitteln eines Haarpflegeprodukts eine zweite Datenverbindung 674 zwischen dem Computer 660 und der Kamera 336 aufweisen. Mittels der zweiten Datenverbindung 674 können Daten vom Computer 660 an die Kamera 336 übermittelt werden, beispielsweise für eine, z.B. herkömmliche, Softwaresteuerung der Kamera 336. Ferner können mittels der zweiten Datenverbindung 674 Daten, beispielsweise das/die von der Kamera 336 aufgenommenen digital/en Bild/er, an den Computer 660 übertragen werden. In einem Fall, dass die Vorrichtung 600 ein Smartphone, einen Tablet o.ä. aufweist, können die Kamera 336 und die zweite Datenverbindung 674 in der Vorrichtung 600 integriert sein.

In verschiedenen Ausführungsbeispielen kann bei der Vorrichtung 600 zum Ermitteln eines Welligkeitswerts von Haaren bzw. zum Ermitteln eines Haarpflegeprodukts auf eine Kamera 336 verzichtet werden, beispielsweise wenn der Datenverarbeitungsvorrichtung 660 das digitale Bild 100 auf andere Weise bereitgestellt wird, beispielsweise mittels einer Datenübertragung.

Die Datenverarbeitungsvorrichtung 660 kann eingerichtet sein, beispielsweise unter Verwendung des Prozessors 662, das von der Kamera 336 oder auf andere Weise empfangene Bild mittels einer Bildverarbeitungssoftware zu verarbeiten, beispielsweise um auf bekannte Weise in dem empfangenen Bild den Haardarstellungsbereich zu ermitteln und, wie oben für verschiedenen Ausführungsbeispiele beschrieben, den Welligkeitswert von Haaren zu ermitteln. Die Bildverarbeitungssoftware kann in verschiedenen Ausführungsbeispielen eine App aufweisen.

In verschiedenen Ausführungsbeispielen kann die Datenverarbeitungsvorrichtung 600 eine Eingabevorrichtung 668 zum Bereitstellen von Informationen an die Datenverarbeitungsvorrichtung 600 aufweisen, beispielsweise eine Tastatur, eine Maus, eine berührungsempfindliche Oberfläche der Anzeigevorrichtung 664, oder ähnliches.

Die Eingabevorrichtung 668 kann mittels einer dritten Datenverbindung 672 mit der Datenverarbeitungsvorrichtung 660 verbunden sein. Die Eingabevorrichtung 668 kann mit der Datenverarbeitungsvorrichtung 660 mittels der dritten Datenverbindung 672 Daten austauschen. In einem Fall, dass die Vorrichtung 600 ein Smartphone, einen Tablet o.ä. aufweist, können die Eingabevorrichtung 668 und die dritte Datenverbindung 672 in der Vorrichtung 600 integriert sein.

Weitere vorteilhafte Ausgestaltungen des Verfahrens ergeben sich aus der Beschreibung der Vorrichtung und umgekehrt.

## Patentansprüche

1. Verfahren zum Ermitteln eines Welligkeitswerts von Haaren, aufweisend:
Aufnehmen mindestens eines digitalen Bildes (100), in welchem Haare (102) abgebildet sind;
während des Aufnehmens des mindestens einen digitalen Bildes, Beleuchten des aufgenommenen Haars mit diffusem Licht (332);
Ermitteln und/oder Festlegen (410) mindestens eines Haaruntersuchungsbereichs (B100: B100_1; B100_2; B100_3) in dem mindestens einem digitalen Bild (100);
Ermitteln (420) einer Mehrzahl von unterschiedlichen Helligkeitswerten in dem Haaruntersuchungsbereich; und
Ermitteln (430; 510) des Welligkeitswerts von Haaren basierend auf der Mehrzahl von Helligkeitswerten, wobei beim Ermitteln des Welligkeitswertes ein Maß für die Heterogenität der Helligkeitswerte der Haare ermittelt wird,
wobei das Aufnehmen des mindestens einen digitalen Bildes aufweist:
Anordnen einer Kamera (336) zum Aufnehmen des digitalen Bildes derart, dass ein Winkel zwischen einer Hauptrichtung, aus der das diffuse Licht das Haar beleuchtet, und einer optischen Achse (340) der Kamera einen Winkel von mindestens 75° aufweist.

2. Verfahren gemäß Anspruch 1,
wobei das Ermitteln einer Mehrzahl von Helligkeitswerten ein Ermitteln eines minimalen Helligkeitswerts (B100_1MIN; B100_2MIN) und eines maximalen Helligkeitswerts (B100_1MAX; B100_2MAX) aufweist; und
wobei das Ermitteln des Welligkeitswerts von Haaren basierend auf der Mehrzahl von Helligkeitswerten ein Ermitteln eines Quotienten des maximalen Helligkeitswerts und des minimalen Helligkeitswerts aufweist.

3. Verfahren gemäß Anspruch 1,
wobei das Ermitteln einer Mehrzahl von Helligkeitswerten ein Ermitteln einer Helligkeitsverteilung (200; 200_1; 200_2) aufweist; und
wobei das Ermitteln des Welligkeitswerts von Haaren basierend auf der Mehrzahl von Helligkeitswerten ein Ermitteln eines Werts für eine Breite der Helligkeitsverteilung aufweist.

4. Verfahren gemäß einem der Ansprüche 1 bis 3,
wobei das Aufnehmen des mindestens einen digitalen Bildes ein Aufnehmen einer Mehrzahl von digitalen Bildern aufweist, und
wobei auf jedem Bild der Mehrzahl von digitalen Bildern Haare aus einem unterschiedlichen Bereich einer Frisur dargestellt sind.

5. Verfahren gemäß Anspruch 4,
wobei das Ermitteln des Welligkeitswerts von Haaren ein Ermitteln eines gemeinsamen Frisur-Welligkeitswerts basierend auf der Mehrzahl von Helligkeitswerten aus der Mehrzahl von digitalen Bildern aufweist.

6. Verfahren gemäß einem der Ansprüche 1 bis 5,
wobei das Festlegen des mindestens einen Haaruntersuchungsbereichs ein Festlegen von Position und/oder Form und/oder Größe des Haaruntersuchungsbereichs aufweist.

7. Verfahren gemäß einem der Ansprüche 1 bis 6,
wobei das digitale Bild in einem Farbraum parametrisiert ist, der eine Helligkeit als einen Parameter aufweist.

8. Verfahren gemäß einem der Ansprüche 1 bis 7, ferner aufweisend:
Darstellen des ermittelten Ergebnisses.

9. Vorrichtung (600) zum Ermitteln eines Welligkeitswerts von Haaren,
aufweisend:
eine Datenverarbeitungsvorrichtung (660); eine Kamera (336); eine Lichtquelle (330); und eine Anzeigevorrichtung (664);
wobei die Vorrichtung eingerichtet ist, das Verfahren gemäß einem der Ansprüche 1 bis 8 auszuführen.

10. Verfahren zum Ermitteln einer individuellen Haarbehandlungsempfehlung, aufweisend:
Ermitteln (430; 510) eines Welligkeitswerts von Haaren gemäß einem der Ansprüche 1 bis 8; und
Ermitteln (520) und Ausgabe einer individuellen Behandlungsempfehlung basierend auf dem ermittelten Welligkeitswert.

## Claims

1. Method for determining a waviness value of hair, comprising:
capturing at least one digital image in which hair is depicted;
while capturing the at least one digital image, illuminating the captured hair with diffuse light (332);
determining and/or defining at least one hair examination area (B100; B100_1; B100_2; B100_3) in the at least one digital image (100);
determining a plurality of different brightness values in the hair examination area; and
determining the waviness value of hair based on the plurality of brightness values, wherein determining the waviness value involves determining a measure of the heterogeneity of the brightness values of the hair,
wherein capturing the at least one digital image comprises:
arranging a camera (336) for capturing the digital image such that an angle between a main direction from which the diffuse light illuminates the hair and an optical axis (340) of the camera is at least 75°.

2. Method according to claim 1,
wherein determining a plurality of brightness values comprises determining a minimum brightness value (B100_1MIN; B100_2MIN) and a maximum brightness value (B100_1MAX; B100_2MAX); and
wherein determining the waviness value of hair based on the plurality of brightness values comprises determining a quotient of the maximum brightness value and the minimum brightness value.

3. Method according to claim 1,
wherein determining a plurality of brightness values comprises determining a brightness distribution (200; 200_1; 200_2); and
wherein determining the waviness value of hair based on the plurality of brightness values comprises determining a value for a width of the brightness distribution.

4. Method according to any one of claims 1 to 3,
wherein the capturing of the at least one digital image comprises capturing a plurality of digital images, and
wherein hair from a different area of a hairstyle is depicted in each of the plurality of digital images.

5. Method according to claim 4,
wherein determining the waviness value of hair comprises determining a common hairstyle waviness value based on the plurality of brightness values from the plurality of digital images.

6. Method according to any one of claims 1 to 5,
wherein determining the at least one hair examination area comprises determining a position and/or shape and/or size of the hair examination area.

7. Method according to any one of claims 1 to 6,
wherein the digital image is parameterized in a color space that has brightness as a parameter.

8. Method according to one of claims 1 to 7, further comprising:
displaying the determined result.

9. Device (600) for determining a waviness value of hair,
comprising:
a data processing device (660); and
a display device (664);
wherein the apparatus is configured to perform the method according to any one of claims 1 to 8.

10. A method for determining an individual hair treatment recommendation, comprising:
determining a wave value of hair according to one of claims 1 to 8; and
determining and outputting an individual treatment recommendation based on the determined waviness value.

## Revendications

1. Procédé pour déterminer une valeur d'ondulation de cheveux, comprenant :
la prise d'au moins une image numérique dans laquelle des cheveux sont représentés ;
pendant la prise de la au moins une image numérique, éclairer les cheveux photographiés avec une lumière diffuse (332) ;
la détermination et/ou la définition d'au moins une zone d'examen des cheveux (B100 ; B100_1 ; B100_2 ; B100_3) dans la au moins une image numérique (100) ;
déterminer une pluralité de valeurs de luminosité différentes dans la zone d'examen des cheveux ; et
déterminer la valeur d'ondulation des cheveux sur la base de la pluralité de valeurs de luminosité, la détermination de la valeur d'ondulation comprenant la détermination d'une mesure de l'hétérogénéité des valeurs de luminosité des cheveux,
la prise de la au moins une image numérique comprenant :
le positionnement d'une caméra (336) pour prendre l'image numérique de telle sorte qu'un angle entre une direction principale à partir de laquelle la lumière diffuse éclaire les cheveux et un axe optique (340) de la caméra présente un angle d'au moins 75°.

2. Procédé selon la revendication 1,
dans lequel la détermination d'une pluralité de valeurs de luminosité comprend la détermination d'une valeur de luminosité minimale (B100_1MIN ; B100_2MIN) et d'une valeur de luminosité maximale (B100_1MAX ; B100_2MAX) ; et
la détermination de la valeur de ondulation des cheveux sur la base de la pluralité de valeurs de luminosité comprenant la détermination d'un quotient de la valeur de luminosité maximale et de la valeur de luminosité minimale.

3. Procédé selon la revendication 1,
dans lequel la détermination d'une pluralité de valeurs de luminosité comprend la détermination d'une distribution de luminosité (200 ; 200_1 ; 200_2) ; et
dans lequel la détermination de la valeur d'ondulation des cheveux sur la base de la pluralité de valeurs de luminosité comprend la détermination d'une valeur pour une largeur de la distribution de luminosité.

4. Procédé selon l'une des revendications 1 à 3,
dans lequel la prise d'au moins une image numérique comprend la prise d'une pluralité d'images numériques, et
dans lequel, sur chaque image de la pluralité d'images numériques, des cheveux provenant d'une zone différente d'une coiffure sont représentés.

5. Procédé selon la revendication 4,
dans lequel la détermination de la valeur d'ondulation des cheveux comprend la détermination d'une valeur d'ondulation de coiffure commune sur la base de la pluralité de valeurs de luminosité provenant de la pluralité d'images numériques.

6. Procédé selon l'une des revendications 1 à 5,
dans lequel la détermination d'au moins une zone d'examen des cheveux comprend la détermination de la position et/ou de la forme et/ou de la taille de la zone d'examen des cheveux.

7. Procédé selon l'une des revendications 1 à 6,
dans lequel l'image numérique est paramétrée dans un espace colorimétrique qui comprend la luminosité comme paramètre.

8. Procédé selon l'une des revendications 1 à 7, comprenant en outre :
la représentation du résultat déterminé.

9. Dispositif (600) pour déterminer une valeur d'ondulation de cheveux,
comprenant :
un dispositif de traitement de données (660) ; et
un dispositif d'affichage (664) ;
le dispositif étant configuré pour exécuter le procédé selon l'une des revendications 1 à 8.

10. Procédé pour déterminer une recommandation individuelle de traitement capillaire, comprenant :
la détermination d'une valeur d'ondulation des cheveux selon l'une des revendications 1 à 8 ; et
la détermination et la délivrance d'une recommandation de traitement individuelle sur la base de la valeur d'ondulation déterminée.
